# EUROPEAN PATENT SPECIFICATION

(11) **EP 2 149 306 B1**
(45) Date of publication and mention of the grant of the patent: **12.02.2014**
(21) Application number: 09166616.4
(22) Date of filing: 28.07.2009
(51) Int. Cl.: A23C 19/02, B01J 13/00, B01J 13/10

(54) **Simultaneous multiple acervation process**
Gleichzeitiges mehrfaches Anhäufungsverfahren
Procédé d'acervation multiple simultanée

(30) Priority: 30.07.2008 US 182674
(43) Date of publication of application: 03.02.2010
(73) Proprietor: Kraft Foods Group Brands LLC, Northfield, IL 60093 (US)
(72) Inventor: Loh, Jimbay Peter, Green Oaks IL 60048 (US); Hong, Yeong-Ching Albert, Kildeer IL 60047 (US); Ma, Yinqing, Arlington Heights IL 60004 (US); Cha, Alice S., Northbrook IL 60062 (US); Kang, Iksoon, Madison Wisconsin 53719 (US)
(74) Representative: Smaggasgale, Gillian Helen

(56) References cited:
- EP-A1- 1 371 410
- EP-A2- 0 626 445
- US-A- 3 639 256
- US-A- 5 492 646

## Description

### FIELD OF THE INVENTION

This invention relates to processes of preparing structured polymer matrices using two or more acervation mechanisms simultaneously.

### BACKGROUND OF THE INVENTION

Acervation is a mechanism or reaction that "heaps up" soluble polymer(s) to form insoluble matrices, structures, or clusters that are significantly greater in size than the individual polymer molecules in solution. Acervation mechanisms include, among others, polymerization, cross-linking, complexing, precipitation (such as isoelectric, ionic, solvent, and the like), coagulation, denaturation (such as by pH, heat, enzyme, chemical, and the like), and coacervation. However, using conventional acervation methods, the characteristics of the solid polymer matrices formed thereby are limited to the particular polymer(s) and/or the particular acervation mechanism(s) used.

For example, enzymatic treatment of casein, a milk protein, with the protease rennin produces an aggregate of very soft structure, whereas treatment of casein with calcium salt forms a dense calcium caseinate precipitate. Adjusting the pH of casein to a pH close to its isoelectric point results in an insoluble, sand-like precipitate commercially known as acid casein. Thus, the particular acervation mechanism used to treat casein in each instance results in acervates (i.e., aggregates or precipitates) having different structural characteristics and textures.

The formation of a semi-solid polymer matrix (such as cheese curd from milk) has commonly been accomplished using a single acervation mechanism. A cheese curd is generally prepared from dairy liquids by processes that include treating the liquid with a coagulating or clotting agent. The coagulating agent may be a curding enzyme (e.g., rennet) or an edible acid, including a suitable bacterial culture to generate edible acrid *in situ*. The resulting coagulum or curd generally incorporates casein that has been altered by the curding process. Generally, the acervation mechanism commonly involved is either enzyme hydrolysis of k-casein or isoelectric precipitation. Although it is common practice to the pH of milk to enzyme addition to reduce the amount of enzyme required, enzyme hydrolysis is the only acervation mechanism involved, as the final curd pH is significantly higher than the isoelectric point of casein. Moreover, enzyme hydrolysis and pH adjustment are not performed simultaneously.

Other single acervation mechanisms are commonly used in the art. Specifically, coacervation of two polymers is commonly used in encapsulation applications. Methods involving second mechanisms generally are used to modify or attempt to modify the structure/acervate formed by the first mechanism. In such cases, the mechanisms are performed sequentially: Littoz et al., Food Hydrocolloids, 22(7): 1203-1211 (2008) (pH adjustment followed by enzymatic crosslinking); Yin et al., J. Macromolecules, 36(23): 8773-8779 (2003) (coacervation followed by crosslinking); Lin et al., J. Pharmaceutical Research, 11(11): 1588-1592 (1994) and Lin et al., J. Biomaterials, 18(7): 559-65 (1997) (producing nanospheres using coacervation followed by crosslinking with glutaraldehyde); and Bachtsi et al., J. Applied Polymer Science, 60(1): 9-20 (1996) (addition of sodium sulfate followed by chemically crosslinking using glutaraldehyde). Use of individual acervation mechanisms or acervation mechanisms performed sequentially result in polymer matrices having characteristics limited to the particular polymer(s), the particular acervation mechanisms used, and/or the order of the particular acervation mechanisms conducted.

The effects of various physical conditions (e.g., temperature, shear, pH, and the like) on specific acervation mechanisms, such as coacervation, phase separation, and cross-linking, have been studied. For example, factors affecting crosslinking or thermal gelation of whey protein have been studied by heating solutions of whey protein at different temperatures, pH, and salt concentrations. Dunkeley & Hayes, J. Dairy Science & Technology, 15:191 (1980) and Xiong, J. Agric. Food Chemistry, 40: 380-384 (1992). However, the physical conditions used in these studies (e.g., pH, salt, and temperature) the kinetics the crosslinking but were insufficient to trigger a simultaneous second acervation mechanism. For example, the pH variations were not sufficiently high to cause substantial polymerization. Moreover, the pH adjustments were applied sequentially or completed prior to the temperature treatment.

In making Ricotta cheese, for example, a mixture of milk and cheese whey is typically acidified by either lactic fermentation or direct addition of food acid (e.g., vinegar) to a pH of about 6.1 prior to a heat coagulation step. The main acervation mechanism used in the Ricotta process is thermal cross-linking of albumin from milk and cheese whey. The acidity is mainly for flavor purposes and, unlike casein, the albumin in whey does not coagulate even at its isoelectric pH. The resulting Ricotta curd is typically small grained and non-cohesive in nature.

U.S. Patent No. 5,952,007 to Bakker et al. is directed to formation of coacervates comprising at least two polymers that are useful as a fat-replacing ingredient. Bakker et al. describes complex coacervation of at least two polymers by heating a solution of the polymers followed by adjusting the pH of the mixture to a value close to the isoelectric point. The mixture is then cooled and the coacervate is isolated. Potentially, there could be more than one acervation mechanism involved but would occur sequentially, if at all.

It would be a significant advance in the art to produce new or improved structured polymer matrices that have not been attainable using single acervation mechanisms or two or more acervation mechanisms performed in series due to the physicochemical properties of the polymer(s) and the acervation mechanisms used, as well as the polymer-polymer interactions involved.

### SUMMARY OF THE INVENTION

The processes described herein provide efficient and economical methods for preparing a structured polymer matrix. The processes described herein avoid the cumbersome and inconvenient sequential treatments of conventional acervation methods. In addition, the processes described herein provide flexible processes for forming structurally complex, polymer matrices from a variety of polymers or combinations of polymers, preferably, although not limited to, food polymers. More preferably, at least one of the polymers is a food protein. The simultaneous application of two or more acervation mechanisms unexpectedly gives structured polymer matrices having improved texture and/or process efficiency that are not attainable when acervation mechanisms are conducted will sequentially. Advantageously, the processes described herein provide great flexibility in the design and production of semi-solid polymer matrices that were not attainable in the past due to unique physicochemical properties of the specific polymers involved, the specific acervation mechanisms used, the specific polymer-polymer interactions involved, and other process limitations. The processes described herein involve two or more acervation mechanisms working simultaneously to form mixed/entangled solid polymer matrices that can be prepared to have a texture ranging from that of a soft and smooth yet cohesive curd to a firm and chewy fiber depending on the combination of acervation mechanisms and polymers selected.

The polymer(s) selected should be capable of forming an aqueous polymer solution before treatment with the simultaneous method described herein. For purposes of the invention, a "polymer solution" or equivalent term includes aqueous solutions where one or more polymer(s) is dissolved, solubilized, or suspended in a finely divided form (preferably in form of a colloidal suspension) such that the polymer(s) can fully react in at least one acervation mechanism. Therefore, the polymer(s) may be treated, such as by adjusting the pH, ionic strength, temperature, and like, if necessary, to prepare the polymer solution.

The concentration of the one or more polymers is selected to provide reliable processing. Because polymers have different molecular weights, different numbers of charged sites at a given pH, the processing conditions for the processes provided herein should be selected such that each polymer used in the reaction is largely consumed by the two or more acervation reactions involved. This ensures that an insignificant amount of either polymer will remain in original soluble form after the simultaneous process. In addition, the final structure/ texture formed may be designed to favor one polymer over the other for the targeted purpose. For example, if the final coacervate is to be used for cheese application, protein may be favored over polysaccharide for nutritional and flavor purposes. The polymer concentration is generally on the order of about 0,01 to about 30 percent by of the solution, although polymer concentrations of about 0.1 to about 10 percent are preferred.

In one aspect, the at least one aqueous solution containing the one or more polymers is capable of undergoing at least two acervation mechanisms. The at least one aqueous solution is formulated and treated so that the conditions are such that the at least two acervation mechanisms are not activated prior to the activation step. The at least one aqueous solution is then treated to activate that at least two acervation mechanisms simultaneously and then the at least two activated acervation mechanisms are allowed to proceed until the structured polymer matrix is obtained.

In another aspect, two or more aqueous polymer solutions are mixed together with or without applying shear such that two or more acervation mechanisms occur instantly and simultaneously. The at least two polymer solutions are formulated such that conditions necessary for each acervation mechanism to occur are provided immediately after the polymer solutions are combined such that the two or more acervation mechanisms occur simultaneously upon mixing to form the desired structured polymer matrix.

The polymers used in the at least one polymer solution may be the same or different polymers. The methods described herein can be performed using a variety of polymers and combinations of polymers. Preferably, the polymers are food polymers. More preferably, at least one of the food polymers is a food protein.

Generally, acervation mechanisms suitable for use in the methods described herein include polymerization, thermal crosslinking, ionic crosslinking, isoelectric precipitation, ionic precipitation, enzymatic crosslinking/coagulation, coacervation, chemical complexing, gelation, solvent precipitation, protein denaturation (such as by pH, heat, enzyme, chemical) and the like. The particular polymers and acervation mechanisms used in the simultaneous method described herein can be selected so as provide a desired texture or to achieve a particular purpose, such as to mitigate undesirable texture defects (e.g., graininess), avoid high energy consuming processing steps (e.g., high shear), allow ingredient saving (e.g., forming structure as polymer concentration), and/or improve functionality (e.g., water holding capacity) as compared to acervated polymeric matrices derived from conventional methods that involve a single acervation mechanism or acervation mechanisms performed sequentially.

### DETAILED DESCRIPTION

The methods described herein are directed to preparing structured polymer matrices by treating at least one polymer solution such that two or more acervation mechanisms occur simultaneously. Preferably, two or more polymer solutions are treated such that two or more acervation mechanisms occur simultaneously upon mixing of the two or more polymer solutions. The simultaneous use of two or more acervation mechanisms unexpectedly gives finely entangled matrices with improved process efficiency and texture that is unique in comparison to polymer matrices produced by acervation mechanisms conducted individually or sequentially. In addition, the methods provided herein provide flexible processes for forming structured polymer matrices from a variety of polymers, preferably, but not limited to, food polymers and, most preferably, at least one of the food polymers is a food protein. It was surprisingly found that performing two or more acervation reactions simultaneously results in unexpected final reaction matrices and that the reaction conditions can be selected so as to provide matrices having desired textural properties. Surprisingly, the matrices resulting from the methods described herein are formed such that neither of the single acervation mechanisms dictates or controls the structure or properties of the resulting matrix. In other words, the matrices formed by the simultaneous methods described herein are unique to those produced by the same acervation mechanisms performed individually or sequentially. The use of simultaneous acervation methods, as described herein, advantageously allows the functional properties of the resulting matrices to be tailored to the particular needs of the application, which would be otherwise difficult or impossibly obtain based on a single acervation reaction or combination of acervation reactions performer in sequence.

In one aspect, the simultaneous method described herein can be used to prepare a mixed/entangled solid polymer matrices with a smooth and cohesive texture resembling fresh cheese curd in both texture and appearance. In another aspect, the simultaneous method described herein can be used to produce fibrous structured polymer matrices.

The polymer(s) selected should be capable of forming an aqueous polymer solution before treatment with the simultaneous method described herein. For purposes of the invention, a "polymer solution" or equivalent term includes aqueous solutions where one or more polymer(s) is dissolved, solubilized, or suspended in a finely divided form (preferably in form of a colloidal suspension) such that the polymer(s) can fully react in at least one acervation mechanism. Therefore, the polymer(s) may be treated, such as by adjusting the pH, ionic strength, temperature, and like, if necessary, to prepare the polymer solution.

Polymers useful in the methods described herein are preferably selected from food polymers, such as proteins, polysaccharides, derivatives thereof, and mixtures thereof. Suitable proteins include vegetable sources, animal sources, yeast-derived sources, and the like. Suitable vegetable sources include legume, pea, grain, bean, nut, cereal, soybean, peanut, oilseed (such as oilseed cottonseed, canola, sunflower seed, and the like), and the like. Suitable animal protein sources include dairy protein, egg protein, fish protein, meat protein, plant-derived protein, microorganism-derived protein, and the like. Suitable meat protein sources include poultry, beef, pork, fish, and other seafood, as well as derivatives such as gelatin, serum albumin, and the like. Suitable dairy protein sources include milk, milk derivatives such as skim milk, milk powder, casein, whey protein, fractionated milk protein, concentrated dairy protein sources, dairy protein isolates, and the like. As used herein, a "concentrated dairy protein source" is a protein source wherein proteins are, or can be reconstituted to be, at a concentration that is greater than the dairy liquid from which they originated. Examples of concentrated dairy protein sources include, but are not limited whey protein concentrate, milk protein concentrate, or a combination thereof. Typically, whey protein concentrate and milk protein concentrate have a protein concentration of at least about 34 percent. Examples of dairy protein isolates include, but are not limited to, whey protein isolate, milk protein isolate, and the like. As used herein, "casein" relates to any, or all, of the phosphoproteins in milk, and to mixtures of any of them. Many casein components have been identified, including but not limited to, α-casein (including αₛ₁-casein and αₛ₂-casein), β-casein, κ-casein, and their genetic variants. As used herein, "whey protein" relates to the proteins contained in the dairy liquid (i.e., whey) obtained as a supernatant of the curds when milk or a dairy liquid containing milk components are curded to produce a cheese-making curd as a semisolid. Whey protein is generally understood to include principally the globular proteins β-lactoglobulin and α-lactalbumin. Whey proteins have high nutritive value for humans, and can provide a favorable sensory quality, conferring a creamy and spreadable quality to dairy products in which they are incorporated. Suitable polysaccharides include xanthan, carrageenan, agar, alginate, carboxymethylcellulose ("CMC"), pectin, starches, locust bean gum, gum tragacanth, gum arabic, gum karaya, gum ghatti, guar gum, cellulose gum, hemicellulose, chitosan, derivatives thereof, and combinations thereof. For polysaccharides, ionic gums such as carrageenan, pectin, alginate, CMC, xanthan, gum arabic, gum karaya, guam ghatti, gellan, agarr chitosan and the like are preferred for their ability to coacervate with protein and undergo ion-induced gelation, particularly where a strong matrix is desired or where low polymer concentration are desired, such as for regulatory (e.g., standard of identity) or economic reasons. In contrast, non-ionic gums such as natural and modified starches, maltodextrin, guar gum, locust bean gum, cellulose, and the like are preferred if a weak structure is desired. Raw or processed food ingredients containing food polymer(s), such as milk, cheese whey, egg, meat slurry, fruit puree, and the like may also be used, if desired.

The combinations of polymers, the concentration of each polymer, and the physicochemical conditions of the at least one polymer solution are selected based on the desired properties of the resulting structured matrix. The properties of the structured matrix, such as texture, can be tailored based on the following factors: (1) the type of polymer or polymers used (e.g., protein, polysaccharide); (2) the number of different polymers; (3) the concentration/ratio of each polymer; and (4) the physicochemical conditions under which the structured matrix is formed, such as pH, ionic strength, temperature, shear/ mixing, and, if at least two polymer solutions are used, the mixing ratio of the polymer solutions. The properties of the structured matrix can also be affected by the type and concentration of setting or crosslinking agent(s), if used, as well as the type and concentration of filler(s), if used.

The two or more acervation mechanisms can be conducted using any method whereby the polymer or polymers, and any required reactants, are treated under conditions suitable for two or more acervation mechanisms to proceed simultaneously. How the two or more acervation mechanisms are activated is not critical so long as the two or more mechanisms are activated in an essentially simultaneous manner (i.e., such that mechanisms and the resulting reactions are occurring at the same time). Thus, one could prepare a single solution containing the one or more polymers and then quickly modify the conditions (e.g., temperature, pH, reagents added, and the like) to activate the two or more mechanisms. For example, a solution can be prepared comprising whey protein and carrageenan at high pH and high temperature (e.g., above the crosslinking temperature of whey protein). Because the whey protein and carrageenan are at the same pH, and thus similarly charged, the two polymers cannot coacervate or crosslink. An acid can be added in an amount sufficient to lower the pH to a target pH of about 5.0, thus triggering coacervation and thermal crosslinking to occur simultaneously.

Alternatively, one could prepare two or more polymer solutions (containing the same or different polymers) under conditions where none of the mechanisms are activated and then mix the two or more polymer solutions under conditions whereby the mechanisms are activated. The individual solutions are adjusted so that the two or more acervation mechanisms are only activated when the separate solutions are mixed together. Especially for laboratory scale experiments, this procedure provides a convenient and effective method to achieve the desired simultaneous acervation reactions. Such a procedure was used for the examples presented herein to characterize the invention. Likewise, the discussion and general characterization herein is generally presented in terms of this separate polymer solutions method for convenience. As one skilled in the art will understand, other methods can be used such that two or more acervation reactions occur simultaneously,

Polymer combinations are selected based on the inherent properties of each polymer. A polymer solution can undergo rapid acervation by itself under a sudden change of physicochemical condition (e.g., temperature or pH) and/or with another polymer through polymer-polymer interaction (e.g., crosslinking, coacervation, or complexing). For example, a hot, acidic, thermally denaturable protein solution (such as at about pH 3.0 and about 80°C) can be mixed with a hot, basic polysaccharide solution (such as at about pH 9.0 and 80°C) to form a mixture having a pH of about 5.0. In this system, a complex and composite matrix can be formed through at least three acervation mechanisms operating simultaneously, namely (1) coacervation between strongly positive charged protein molecules and strongly negative charged polysaccharide molecules, (2) isoelectric precipitation of the protein, and (3) covalent crosslinking of protein molecules through heat induced covalent (e.g., disulfide) bond formation. Prior to mixing the two hot polymer solutions, thermal crosslinking of the proteins molecules at low pH is inhibited due to electrostatic repulsion between the protein molecules, as is evident by a clear or translucent protein solution. Crosslinking is no longer inhibited when the solution is mixed with the basic polysaccharide solution and the pH of the mixture is neutralized. Thus, by varying (1) the concentration of one or both of the polymers involved, (2) the ratio of the two polymer solutions, (3) the target pH after mixing the two solutions, and (4) the solution temperature after mixing the two solutions, one can rapidly form a non-homogenous, mixed, intertwined, and/or composite structure having a texture varying from soft to firm and appearance varying from curd-like to fibrous. Staining the matrix formed with protein specific and polysaccharide specific dyes can be used. Use of such dyes to visually view at least some aspects of the simultaneous coacervation mechanisms suggests that molecular exclusion does not occur significantly or fast enough due to the rapid, simultaneous multiple acervation reactions. Although not wishing to be limited by theory, the staining suggests that the polymers form inseparable complexes (e.g. particles, fibers, and the like) and not separate acervates from each polymer.

In the methods described herein, the polymer concentration is about 0.01 to about 30 percent by weight of the solution although polymer concentrations of about 0.1 to about 10 are preferred. In one aspect, a solution containing one or more polymers is treated such that two or more acervation mechanisms occur simultaneously to form a structured polymer matrix. In another aspect, two or more polymer solutions are combined such that at least two acervation mechanisms occur simultaneously upon mixing to form a structured polymer matrix. In another aspect, at least three polymer solutions are treated and combined such that, upon mixing, the two or more acervation mechanisms occur simultaneously to form a structured polymer matrix. In another aspect, at least two polymer solutions are combined and treated such that three or more acervation mechanisms occur simultaneously upon mixing to form a structured polymer matrix. The polymers in the two or more solutions may be the same or different polymers.

Generally, acervation mechanisms that can be used in the simultaneous method described herein include polymerization, thermal crosslinking, ionic crosslinking, isoelectric precipitation, ionic precipitation, enzymatic coagulation, coacervation, chemical complexing, isoelectric precipitation, ionic precipitation, gelation, solvent precipitation, denaturation (such as denaturation of protein by pH, heat, enzyme, and chemical treatment), and the like. A complex, mixed, intertwined or composite structure is formed that may or may not be microscopically homogeneous and the polymer(s) used may not be individually discernable if both polymers are nearly completely consumed in the process.

The particular selection of two or more acervation mechanisms based on the desired characteristics of the resulting structured matrix will readily be within the capability of one of ordinary skill in the art. The selection of acervation mechanisms depends on the particular combination of polymers selected as well as the desired characteristics of the resulting structural matrix. In addition, one of ordinary skill in the art will recognize that not all combinations of polymers and/or all acervation mechanisms will be suitable or even feasible to allow simultaneous reaction. For example, thermal crosslinking is a commonly used mechanism for whey protein while enzyme coagulation is a commonly used acervation mechanism for casein. However, to engineer a process to allow both acervation mechanisms to occur simultaneously and efficiently is not always possible, as enzyme coagulation generally proceeds slowly over time. For example, milk, a natural mixture of both casein and whey protein, can be heated to crosslink whey protein and then cooled prior to the addition of coagulating enzyme to form casein curd. Alternatively, milk can be treated with coagulating enzyme and then heated. However, neither method provides for simultaneous acervation mechanisms. Thermal crosslinking of whey protein is a kinetic process that highly depends on whey protein concentration, pH, heating temperature, and heating time, whereas the enzyme coagulation of casein, also a slow kinetic driven process, can only occur below the minimum crosslinking temperature of whey protein. To create a process that would allow both acervation mechanisms to occur simultaneously does not appear to be feasible since the two mechanisms require significantly different temperatures to proceed and enzyme coagulation would take much longer than thermal crosslinking. As another example, where one polymer solution comprises whey protein isolate and the other polymer solution comprises casein, simultaneous acervation mechanisms including both thermal crosslinking and enzyme coagulation are practically impossible. However, because casein can undergo acervation by mechanisms other than enzyme coagulation, such as isoelectric precipitation, a simultaneous multiple acervation process may be designed using mechanisms other than enzyme coagulation. For example, a first polymer solution comprising whey protein isolate at a pH of about 3.5 and a temperature of about 82.2°C (180°F) and a second polymer solution comprising caseinate at a pH of 8.0 and a temperature about 82.2°C (180°F) can be mixed to provide a final, equilibrium pH around 4.6 where at least two acervation reactions will occur simultaneously-thermal crosslinking of the whey protein and acid precipitation of caseinate. Thermal crosslinking of the whey protein molecules at low pH is inhibited due to electrostatic repulsion between the protein molecules, as is evident by a clear or translucent protein solution. Crosslinking is no longer inhibited when the solution is mixed with the caseinate solution and the pH of the mixture is neutralized. Thus, the selection of polymers and combination of acervation mechanisms depends on the physical limitations inherent to each polymer and acervation mechanism selected.

The simultaneous method described herein a flexible process for performing multiple acervation reactions simultaneously by the polymer(s), acervation mechanisms, and physicochemical conditions such that structured polymeric matrices are formed that were not previously achievable by carrying out the same acervation reactions alone or in sequence. While not intending to be limiting, several acervation mechanisms are described in greater detail.

### Coacervation

Coacervation generally involves combining two oppositely charged polymers in solution to bring about separation of an insoluble complex or coacervate. In coacervation, two oppositely charged polymer solutions are prepared. Preferably, a positively charged protein solution and a negatively charged polysaccharide solution. Alternatively a positively charged complex polysaccharide solution (such as chitosan) and a negatively charged protein or another polysaccharide solution can be used, if desired. An aqueous solution of a first polymer is prepared and the pH is adjusted to about 2 to about 5 using a food grade acid to form a positively charged polymer. Suitable food grade acids include, but are not limited to, phosphoric acid, hydrochloric acid, sulfuric acid, lactic acid, citric acid, and combinations thereof. Because the first polymer is predominantly positively charged, the like-charged molecules exhibit repulsive forces. An aqueous solution of a second polymer is prepared and the pH is adjusted to about 8 to about 11 using a food grade base to form a negatively charged polymer. Suitable food grade bases include, but are not limited to, sodium hydroxide, potassium hydroxide, calcium hydroxide, and combinations thereof. Because the second polymer is predominantly negatively charged, the like-charged molecules exhibit repulsive forces and the polymer remains soluble in solution. The oppositely charged polymer solutions are then combined and the oppositely charged polymers attract each other, thus forming an insoluble, mixed polymer matrix before the ionic charges of the first and second polymers are neutralized. The reactions forming the mixed polymer matrix are generally irreversible, although they may be reversible under extreme conditions, such as very high or very low pH. However, under typical or most end use conditions (such as in food products), the formation of the mixed polymer matrix is irreversible.

Coacervation can also be provided by preparing a mixed solution of two polymers (typically a soluble protein and an ionic polysaccharide), followed by titrating the solution pH to slightly below the isoelectric pH of the protein to induce coacervation. At such pH, the protein becomes predominantly positively charged whereas the ionic polysaccharide is negatively charged and an insoluble matrix is formed due to intermolecular static attraction.

As defined herein, self-acervation is a specific type of coacervation. In self-acervation, the first and second polymers used in coacervation are the same polymer. Self-acervation is generally limited to amphoteric polymers, preferably amphoteric proteins. For example, the positively-charged first polymer solution and the negatively-charged second polymer solution both comprise whey protein isolate. If desired, other polymers may be used in self-acervation mechanisms as well. Some polysaccharides may not be suitable for self-acervation mechanisms. For example, anionic polysaccharides that only exist in a neutral or negatively charged form but never in positively charged form would not be suitable. Thus, anionic polysaccharides generally cannot undergo self-acervation as described herein. Among food polymers, only proteins and proteinaceous material, such as ground meat and milk are known amphoteric polymers. Other amphoteric molecules are often non-edible or have low molecular weight, which have little or no potential for structure forming.

In some cases, the acervates formed by coacervation may have properties that are undesirable, thereby limiting their use in food products. In such cases, a second or even a third acervation mechanism may be incorporated to provide simultaneous, multiple acervation reactions to achieve a structured matrix. Indeed, by varying the polymer(s), conditions, and acervation mechanisms, it may be possible to provide structure matrices having useful properties not currently possible in the food industry.

### Thermal Crosslinking

In thermal crosslinking, polymerization of polymer molecules is induced by heat treatment. For example, when food proteins rich in sulfur-containing amino acids are heated at sufficiently high temperature, inter-polymer covalent -S-S- bonds are formed between -SH groups located in different polymer molecules and result in the formation of insoluble polymeric matrices or particles. Food proteins rich in sulfur-containing amino acids include, but are not limited to, whey protein, egg protein, vegetable protein, and the like. Thermal crosslinking of whey protein is a kinetically controlled reaction and the degree of crosslinking is influenced by protein concentration, temperature, heating time, and pH. Because thermal crosslinking can be prevented or substantially reduced at a solution pH significantly different (e.g., two or more pH units) from the isoelectric pH of the polymer, thermal crosslinking of whey protein is a desirable acervation mechanism for providing a simultaneous multiple acervation reaction with at least one other polymer and acervation mechanism. The reaction conditions should be selected so as to minimize the amount of crosslinking that occurs prior to mixing with one or more other polymer solutions. For example, crosslinking of whey protein can be substantially reduced (e.g.., less than about 30 percent) at a pH below 3.5 at a temperature of about 90° C for extended heat treatments, such as for about 30 minutes. Alternatively, the solution containing the crosslinkable polymer may be maintained at a lower temperature and the other solution (the solution not containing crosslinkable polymer) could be heated to a temperature such that, when mixed, the temperature of the mixture is suitable for the crosslinking reaction. Conditions for other activation methods and/or polymers can be readily selected by those of ordinary skill in the art.

### Simultaneous Coacervation and Thermal Crosslinking

In order to provide a simultaneous multiple acervation process that allows coacervation between a first polymer and a second polymer to coincide with the thermal crosslinking of the first polymer, the first polymer should be capable of forming crosslinks at or above a temperature provided upon mixing of solutions containing the two polymers. The first and second polymers must be oppositely charged to allow coacervation between the polymers. To do so, an aqueous solution of a first polymer is prepared and the pH is adjusted to a pH sufficiently lower than the isoelectric pH of the first polymer (e.g., pH of about 2 to 5) using a food grade acid to form a positively charged polymer solution. Because the first polymer is predominantly positively charged under low pH conditions, the like-charged first polymer molecules exhibit repulsive forces and remain soluble in the highly acidic solution. The intermolecular repulsive forces also allow the highly acidic solution to be heated to a temperature equal or higher than the crosslinking temperature of the first polymer at a normal or less acidic pH (e.g., less than about 1 pH unit lower than the isoelectric pH of the first polymer) without inducing significant crosslinking. Advantageously, the first polymer solution remains clear or translucent in appearance as the polymers stay soluble and non-acervated in the hot and highly acidic solution. A second polymer solution is prepared by dissolving the second polymer in water and the pH of the solution is adjusted to a pH sufficiently higher than the isoelectric pH of the second polymer (e.g., pH of about 8 to 11) using a food grade base to form a negatively charged second polymer solution. Because the second polymer is predominantly negatively charged, the like-charged molecules also exhibit repulsive forces and remain soluble in the highly basic solution, even at an elevated temperature. The negatively-charged second polymer solution is heated to a temperature equal to or greater than the temperature of the first polymer solution. Because the cross-linkable polymers are like-charged in their respective solutions and exhibit repulsive forces, the polymers remain substantially uncrosslinked even under relatively severe heat treatment. Generally, the acidic and basic polymer solutions are heated to a temperature ranging from about 65.6 to 93.3°C (150 to about 200° F), preferably about 76.7 to 85°C (170 to about 185° F) prior to mixing the two solutions.

The pH of the two solutions and the ratio at which the two solutions are combined are selected based on the targeted final pH upon mixing the two polymer solutions. The targeted final pH should be a pH at which thermal crosslinking of the first polymer occurs freely, quickly, and effectively, as well as being the pH at which the coacervation of the two polymers can occur. For example, if the first polymer is whey protein isolate and the second polymer is carrageenan, a suitable target pH should be in the range of about 4.2 to about 5.2. In principle, the heating step can, if desired, be conducted at elevated pressures, such as in a heated extruder, in which case the temperature can be suitably adjusted. Without allowing the polymer solutions to cool, the two oppositely charged polymer solutions are mixed, which triggers at least two reactions to occur nearly instantaneously: (1) coacervation: a complex comprising the first and second polymers is formed due to the electrostatic attraction between the two oppositely charged polymers; and (2) crosslinking: the pH of the mixture is neutralized, thus resulting in the removal of repulsive forces between the initially like-charged polymers and resulting in crosslinking between the first polymer molecules through disulfide covalent bonding. The polymerized polymer results from the crosslinking of unfolded proteins by -S-S- bonding. In general, the consequent increase in molecular weight indicates increased crosslinking with the polymer. In principle, at least about 50 percent disulfide crosslinking may be attainable, although crosslinking in the range of about at least 80 percent is generally preferred. The degree of crosslinking can be estimated, for example, using polyacrylamide gel electrophoresis with disulfide reducing agents such as dithiothreitol (see, e.g., U.S. Patent No. 4,885,183 and Laemmi, Nature, 227: 680-685 (1970), both of which are incorporated herein by reference in their entirety). The resulting mixed structured polymer matrix comprises crosslinks of the first polymer and coacervates of the first and second polymer. The structured polymer matrix can be cooled to refrigeration temperature and stored for later use, or the structured polymer matrix can be further processed immediately after matrix formation for incorporation into food products, such as for addition to a cream cheese stream as a texture builder or fat mimetic.

The simultaneous use of coacervation and thermal crosslinking provides several benefits. Lower polymer concentrations can be used when prepare the matrix using the simultaneous method. For example, using a conventional crosslinking mechanism by itself, a 5 to 8 percent whey protein solution is heated for about 30 to 60 minutes at an elevated temperature to form crosslinked whey protein. In contrast, the simultaneous, multiple acervation process described herein requires less than 3 percent whey protein and forms a structured matrix nearly instantaneously. The simultaneous method is also more efficient and less energy intensive process than other methods. Generally, in conventional crosslinking mechanisms, constant high shear or mixing is required to control the particle size of the crosslinks. Numerous unit operations are conventionally used to produce crosslinked whey protein of desirable particle size and off-flavors are often generated due to prolong high temperature exposure. Moreover, producing whey protein is is expensive and energy intensive because the whey protein concentration in cheese whey is extremely low (lower than 0.5 percent) and must be concentrated and extracted, such as by ultrafiltration.

### Isoelectric Precipitation

The isoelectric point is the pH at which the net charge of the polymer, especially amphoteric polymers, is zero. Isoelectric precipitation occurs when polymer molecules in solution at or near the polymer's isoelectric point become insoluble and/or collapse on each other due to the lack of electrostatic stabilization and increased intra-and inter-molecular hydrophobic forces. Isoelectric precipitation can be initiated by adjusting solution pH or ionic strength. For example, isoelectric precipitation is commonly used for making commercial caseinate from milk by adjusting the milk pH with an edible acid to the isoelectric pH of casein (about 4.6). Insoluble casein forms a precipitate or dense curd and can be readily separated from the remaining whey. Isoelectric precipitation can be used in the design of a simultaneous multiple coacervation process.

### Ionic Precipitation

Ionic precipitation of polymers typically involves ionic crosslinking among negatively charged polymer molecules in the presence of multivalent cationic mineral ions. Many food polymers, including most food proteins and most anionic food polysaccharides, can undergo ionic precipitation at a pH greater than their respective isoelectric pH. Anionic food polysaccharides include, but are not limited to, carrageenan, xanthan, alginate, agar, carboxylmethylcellulose, low methoxy pectin, gellan, agar, the like, and mixtures thereof. For example, when a solution of divalent cations (such as CaCl₂) is added to a solution of a negatively charged polymer (such as alginate), calcium bridges are formed between adjacent alginate molecules. Generally, about 0.01 percent divalent cations is used. Depending on the relative concentrations of the ingredients and the physical conditions, the neutralization of the polysaccharide's negative charge by the positively charged calcium ions and formation of calcium bridges between alginate molecules causes precipitation and/or gelation of the polymer. Theoretically, ionic precipitation can also take place among positively charged polymers and suitable anions (e.g., phosphate ions), but this type of ionic precipitation is less common in food systems.

Related mechanisms can be provided by altering the concentration of the polysaccharide and/or mineral cations. A low concentration of Ca²⁺ cations causes a dilute alginate solution to gel. In contrast, a high concentration of Ca²⁺ cations causes complete neutralization of the polysaccharide and precipitation of insoluble, dense calcium alginate.

### Simultaneous Isoelectric Precipitation and Ionic Precipitation

Isoelectric precipitation of one polymer and ionic precipitation of another polymer performed simultaneously to form a mixed, structured polymer matrix is possible based on the inventive process. Generally, two or more polymer solutions are prepared. A first polymer solution is adjusted to a pH of about 8 to about 10 using a food grade base to form a negatively charged polymer solution. A second polymer solution is adjusted to about pH 3 to about pH 4 using a food grade acid to form a positively charged polymer solution. Multivalent mineral cations containing salt are added to either of the polymer solutions, such as about 0.01 percent cations. The pH of the first and second polymer solutions, as well as the mixing ratio thereof, are selected such that the final pH after mixing the two solutions is near the isoelectric point of the polymer to be precipitated. Upon mixing the two polymer solution, several changes happen simultaneously, including: (1) neutralization of the mixture to a pH substantially near the isoelectric pH of the polymer to be precipitated; (2) isoelectric precipitation of the first polymer; and (3) ionic precipitation of the second polymer with the mineral cations. As a result, a mixed structured matrix is formed which has a curd-like appearance and texture different than matrices produced by single acervation or sequential acervation mechanisms performed with the same polymer(s). For example, textural differences can include sensorial (e.g., appearance, mouthfeel, and the like), physical (mechanical, density, and the like), and functional differences (e.g., water holding capacity).

As will be readily apparent to one of ordinary skill in the art, various combinations of acervation mechanisms can be carried out simultaneously to form structured polymer matrices having the desired textural, physical, and functional properties. As is also readily apparent, various methods of simultaneously activating the acervation mechanisms, other than those given above, can also be used. It was surprisingly found that conducting two or more acervation mechanisms simultaneously results in a structured polymer matrix that is unique and superior to that formed as a result of the same two or more acervation mechanisms conducted alone or sequentially. While every possible combination of mechanisms and/or polymers cannot be discussed herein, it is believed that the simultaneous multiple acervation process described herein applies to a wide range of acervation mechanisms and polymer combinations.

If desired, one or more optional fillers can be added to one or more of the polymer solutions. The fillers should be selected so as not to substantially interfere with the structure formation of the intended polymer matrix. The fillers can be added at about 0 to about 70 percent based on the final matrix. The fillers herein are defined as edible ingredients that are substantially inert or non-reactive food ingredients and functionally act as a structural spacer. The filler is selected from, but not limited to, natural or modified starch, maltodextrin, starch or grain derivatives (e.g., corn syrup solids, rice bran), alpha cellulose, microcrystalline cellulose, fiber, denatured protein (e.g., lactoalbumin, buttermilk solid, and the like), neutral gums (e.g., locus bean gum, guar gum and the like), lipid, and mixtures thereof. Inert fillers are used herein mainly to modify the ultimate matrix structure formed by the simultaneous multiple acervation process. For example, buttermilk solid, a non-reactive, denatured milk membrane protein from butter, does not contribute substantially to the texture during the matrix structure formation of a finished food product (e.g., cream cheese) but can be incorporated into or structurally entrapped in a co-polymeric matrix structure of two other polymers formed using the simultaneous method described herein. Practical benefits of incorporating fillers can vary and include, but are not limited to, increased yield/volume, increased water holding capacity, reduced density, reduced firmness/toughness, and improved of the final polymer matrix or the final food product containing the polymer matrix.

If desired, optional ingredients, such as emulsifier, salt, sweetener, acidulant, colorant, flavor, stabilizer and the like, can be added in either one or both of the at least two polymer solutions at a total level of about 0 to about 10 percent so as not to substantially interfere with the structure formation of the intended polymer matrix. Flavorings include, for example, butter flavor, milk flavor, cheese flavor, meat flavor, seasonings, herbs, and fruit or vegetable purees or powders. Colorants such as, for example, β-carotene, annatto, artificial food color, and the like may also be used. Suitable stabilizers include, but are not limited to, antioxidants, antimicrobials, and the like. These optional ingredients should be selected so as not to substantially interfere with or otherwise affect the formation of the structured polymer matrix in an adverse manner, but may be selected so as to interfere in a beneficial manner.

The structured polymer matrix produced by the simultaneous methods described herein can be used directly in a food product or can be recovered from the reaction mixture using any suitable method, such as by centrifugation, filtration, or the like, and then used in a food product, if desired. The structured polymer matrix produced by the simultaneous methods described herein may be used in the production of cheese, such as cream cheese, natural cheese, cheese-like products, meat products or analogs, soy products (such as textured soy products), sauces, dressings, desserts, confections, bakery fillings, or the like.

The following examples describe and illustrate the processes and products of the invention. These examples are intended to be merely illustrative of the present invention, and not limiting thereof in either scope or spirit. Those skilled in the art will readily understand that variations of the materials, conditions, and processes described in these examples can be used. All references cited herein are incorporated by reference in their entirety. Unless otherwise noted, all percentages are by weight of the noted composition.

### EXAMPLES

### Example 1

This example demonstrates the important of performing simultaneous acervation reactions (inventive) versus various control reactions where one acervation reaction is performed at a time. Two solutions were prepared. Solution A included 3 percent whey protein isolate (Bipro from Davisco Foods International, Inc., Le Sueur, MN) in DI water and was pH adjusted to 3.45 with 5N HCl. Solution B included 0.3 percent pre-dissolved carrageenan (Gelcarin GP 911 from FMC Corp., Philadelphia, PA) in DI water and was pH-adjusted to 11.55 with 5N NaOH. Four experiments were conducted to demonstrate the importance of simultaneous acervation reactions.

Control A: Equal amounts of solution A and solution B were mixed at room temperature and allowed to react for at least 10 minutes. Control A demonstrates typical coacervation of carrageenan and whey protein.

Control B: Equal amounts of solution A and solution B were mixed at room temperature and allowed to react for at least 10 minutes and then heated to 82.2°C (180°F) to thermally cross-link the whey protein. After coacervation, the pH of the mixture allows crosslinking. The mixture was then allowed to cool under ambient condition in a sealed glass jar. Control B demonstrated coacervation of carrageenan and whey protein and thermal cross-linking of whey protein performed in sequence.

Control C: Equal amounts of solution A and solution B were separately heated to 82.2°C (180° F) and allowed to cool under ambient condition in a sealed glass jar. Due to the pH of the solutions, little or no cross-linking occurred (i.e., 30 percent or less). After cooling to ambient, solutions A and B were mixed together and allowed to react for at least 10 minutes. Control C demonstrated a different variation of coacervation with a small amount of cross-linking performed in in sequence.

Inventive: Equal amounts of solutions A and B were separately heated to 82.2°C (180° F). Immediately after reaching the targeted temperature, solutions A and B were mixed together and allowed to cool under ambient condition in a sealed glass jar. This process resulted in simultaneous coacervation and thermal crosslinking.

The quantity of curds generated in each experiment was measured by filtration. Each sample from the experiments described above was passed through a US50 sieve and the mass retained on top of the sieve was measured. The results are provided in Table 1 below.

**Table 1. Effect of various control and experimental treatment on curd formation.**

| | Control A | Control B | Control C | Inventive |
|---|---|---|---|---|
| % Curd | 8.1% | 12.9% | 9.0% | 27.9% |

The inventive sample from the inventive simultaneous reaction had significantly more curds than that formed by single coacervation (control A) or coacervation and thermal cross-linking in sequence (control B and control C).

### Example 2

To further demonstrate the importance of simultaneous reactions, another set of experiments was conducted at various reaction temperatures. Four separate solutions each of solution A and B prepared as described in Example 1 were heated to 54.4, 65.6, 76.7, and 82.2 °C (130°F, 150°F, 170° F, and 180° F) respectively. Immediately after heating to the targeted temperature, equal amounts of solution A and solution B at the same temperature were mixed together (i.e., solution A at 54.4°C (130°F) was mixed with solution B at 54.4°C (130°F), solution A at 65.6°C (150°F) was mixed with solution B at 65.6°C (150°F), and so on). Each mixture was then allowed to cool under ambient condition in a sealed glass jar. The quantity of curds generated was measured by filtration, as described above, and the results are provided in Table 2 below.

**Table 2. Effect of reaction temperature on curd formation.**

| | Reaction Temperature | | | |
|---|---|---|---|---|
| | 54.4°C (130° F) | 65.6°C (150° F) | 76.7°C (170° F) | 82.2°C (180° F) |
| % Curd | 9.4% | 9.6% | 21.1% | 27.9% |

The solutions at 54.4°C (130°F) and 65.6°C (150°F) formed significantly lower amounts of curds compared to the solutions mixed at 76.7°C (170°F) and 82.2°C (180°F). This is believed to be because minimal thermal cross-linking of whey protein occurs at 65.6°C (150° F) and below. The samples prepared with solutions at 76.7°C (170°F) and with solutions at 82.2°C (180°F) both demonstrate simultaneous coacervation and thermal cross-linking reactions. It is believed that the samples prepared with solutions at 54.4°C (130°F) and 65.6°C (150°F) demonstrate mostly coacervation reaction. This experiment further demonstrates the uniqueness of having multiple reactions simultaneously.

### Example 3

Method Using Two Acervation Mechanisms Performed in Series With Different Polymers. Thermal crosslinking and coacervation between whey protein isolate (WPI) and milk protein concentrate (MPC): Acidic (pH=3.45) WPI and basic MPC (Nutrilac 7318, Aria Foods Ingredients, NJ) solutions were prepared by mixing 10 percent protein powder in DI water and adjusting the solution pH to 3.45 and 8.0 using food grade HCl and NaOH, respectively. The two protein solutions were heated in a microwave oven to a temperature of about 90°C. Both heated solutions remained transparent in appearance, which indicates that the proteins were likely not significantly altered in microstructure. The two hot solutions were mixed together immediately (i.e., within about 60 seconds) after reaching 90°C at a ratio of 1:1.

This experiment demonstrates that two acervation mechanisms (thermal crosslinking of whey protein and coacervation between two different proteins) were carried out simultaneously to form a soft, smooth and cohesive curd that resembled normal, fresh cheese curd in texture and appearance. Aided by co-acervation between the 2 different and oppositely charged protein molecules, thermal crosslinking of whey protein became possible at local pH.

### Example 4

This example the different effects of two acervation mechanisms in series versus performing the same two acervation mechanisms simultaneously.

Inventive example. This experiment illustrates an embodiment of the invention where two acervation mechanisms (thermal crosslinking of whey protein and co-acervation between a positively charged protein and a negatively charged polysaccharide) were carried out simultaneously to form a soft, smooth and cohesive curd. An acidic solution of whey protein isolate was prepared by mixing 10 percent whey protein isolate in DI water and adjusting the pH to 3.45 using food grade HCl. A basic solution of xanthan was prepared by mixing 0.5 percent xanthan (Keltrol 519 from CP Kelco) in DI water and adjusting the pH to 11.6 using food grade NaOH.

The two oppositely charged polymer solutions were heated in a microwave oven to a temperature of about 90°C. Additional 5N NaOH was added to the basic xanthan solution in an amount effective to provide a pH of about 5.5 upon mixing with the acidic whey protein solution. Both heated solutions remained transparent in appearance, thus indicating that the polymers were not significantly altered in microstructure by the heat treatment. The two heated polymer solutions were immediately mixed together after heat treatment at a ratio of whey protein isolate solution to xanthan solution of 2.5:1. The mixture formed a full body curd having a pH of about 5.7.

Comparative example. An acidic solution (pH 3.45) of whey protein isolate and a basic solution of xanthan (pH 11.6) were prepared as described above. The two oppositely charged solutions were mixed at room temperature without prior heat treatment. The resulting mixture formed a milky dispersion of relatively high fluidity (presumably from coacervation) but no curd was formed after holding at ambient temperature for about 20 minutes. This mixture was further subjected to heat treatment at 90°C for 2 minutes and again failed to form a cohesive curd. The mixture was allowed to cool and had not formed a curd after 24 hours of storage at refrigeration temperature. While not wishing to wound by theory, it is believed that coacervation of whey protein with a significant amount of xanthan impaired the crosslinking ability of whey protein.

The results of this experiment indicate that simultaneous acervation mechanisms are effective for a wide range of polymers.

### Example 5

This example illustrates how performing multiple acervation mechanisms simultaneously (ionic crosslinking and isoelectric precipitation) results in a structured matrix very different from the product of a single acervation mechanism (isoelectric precipitation) using alginate and milk protein concentrate as the polymers.

Inventive Example. An acidic alginate solution was prepared by mixing DI water with 1 percent alginate (Kimica Corp., Japan) and adjusting the pH to 3.0 using food grade HCl. A basic milk protein concentrate solution was prepared by adding a few drops of 0.1 M CaCl₂ solution to 10 percent milk protein concentrate (Nutrilac from Arla Foods) in DI water, followed by adjusting the pH to 8.2 using food grade NaOH. The two solutions were mixed together at a 3:1 ratio of alginate solution to milk protein concentrate solution at room temperature without any heat treatment to provide a final pH of 4.8. The isoelectric point pH's of caseinate and whey protein are about 4.6 and 5.1, respectively, and both caseinate and whey protein became largely insoluble at pH 4.8. The CaCl₂ caused the alginate to ionically crosslink upon mixing while isoelectric precipitation of milk proteins simultaneously occurred. The resulting mixture had a curd-like texture with clear evidence of syneresis (whey separation).

Comparative Example. An acidic alginate solution and a basic milk protein concentrate solution were prepared as above except for no CaCl₂ was added to the basic milk protein solution. The two solutions were mixed together at a 3:1 ratio of alginate solution to milk protein concentrate solution at room temperature without any heat treatment to provide isoelectric precipitation of the milk proteins. No ionic crosslinking occurred. Alginate is not an amphoteric polymer and is not charged at pH 3.0. Therefore, coacervation between milk proteins and alginate is believed to be unlikely. The resulting mixture was opaque and fluid without curd formation.

This example further demonstrates that the inventive process is generally applicable to combinations of non-thermal acervation mechanisms.

### Example 6

100 Percent Whey Protein Cheese. Many cheeses commonly referred to as "whey cheeses," such as Ricotta cheese, are in fact commonly made from casein (>75 percent in the form of whole milk) and merely supplemented with cheese whey to allow curd-flake formation at high temperature (instead of using rennet). This example demonstrates that simultaneous use of three acervation mechanisms can be used to prepare a 100 percent whey protein (casein-free) hard cheese.

Ten percent whey protein isolate (BiPro from Davisco Foods International, Inc., La Sueur, MN) solution was first prepared, divided and pH-adjusted to provide acidic (pH 3.5) and basic (pH 8.5) whey protein isolate solutions, respectively. Based on protein content, equal amounts of anhydrous milk fat (AMF) was added to the whey protein isolate solutions and homogenized in a lab blender at about 62.8°C (145°F) to form whey protein isolate emulsions. The resulting whey protein isolate emulsions were heated in a microwave oven to a temperature about 87.8°C to 98.9°C (190 to 210°F). The two heated whey protein isolate emulsions were gently mixed together (with a spatula for 10 seconds) in a container. After about 10 minutes of dwell time, the mixture was poured in a strainer with cheese cloth to separate curd (about 64 percent) from whey (about 36 percent). The curd was further salted with 2.5 percent (based on weight of curd) table salt and packed/ pressed in a mold for 2 hours to form a block cheese. After refrigeration for about 12 hours, the resulting 100 percent whey cheese was evaluated by a small expert panel and was determined to be of acceptable flavor, taste, texture and appearance similar to those of casein-based fresh Mexican cheese. In this example, the whey protein molecules were believed to simultaneously undergo self-acenration, isoelectric precipitation, and thermal crosslinking.

### Example 7

Cream Cheese with Increased Firmness. A cheese curd-like complex was prepared using simultaneous multiple coacervation reactions according to the following formula and procedure and then evaluated in a low fat cheese model.

Solution "A1" was prepared by mixing 7 percent whey protein isolate (Bipro) in DI water and adjusted to pH 3.5 using 88 percent lactic acid. Solution A1 was then heated in a microwave oven to 73.9°C (165°F) followed by immediate cooling to room temperature. Solution "A2" was prepared by mixing 7 percent whey protein isolate (Bipro) in DI water and adjusted to pH 3.5 using 88 percent lactic acid and then heated to 90.6°C (195°F) in a microwave oven. Solution A2 was maintained at 87.8 to 90.6°C (190 to about 195°F) to keep the solution hot but not boiling for 20 minutes prior to cooling to below ambient temperature. Solution "B1" was prepared by mixing buttermilk solids (25 percent) and carboxymethylcellulose (0.16 percent) in whole milk and pH adjusted to pH 8.5 using 6N NaOH. Solution "B2" was prepared by mixing buttermilk solids (25 percent) and carrageenan (0.16 percent) in whole milk and pH adjusted to pH 8.5 using 6N NaOH.

Solutions A1 and A2 were then mixed together at a ratio of 43.7 to 8.3, respectively, to form solution A mixture, as shown in Table 3 below. Separately, solutions B1 and B2 were mixed together at a ratio of 33.3 to 16.7, respectively, to form solution B mixture. One part of the solution A mixture and one part of the solution B mixture were separately heated to 87.8°C (190°F). Additional 88 percent lactic acid was added to the hot mixture of the A solutions to target a final complex pH of 5.1. The hot B-solution mixture was gently mixed with the hot A-solution mixture in a Thermomix to form a complex. The complex was allowed to cool to room temperature and stored in the refrigerator prior to use for cream cheese preparation. A soft cream cheese curd-like complex was obtained.

**Table 3.**

| **Ratio** | Solution A1 | Solution A2 | Solution B1 | Solution B2 |
|---|---|---|---|---|
| | 43.7 | 8.3 | 33.3 | 16.7 |
| | | | | |

| **Composition of Complex** | | Amount | Protein | Carrageenan +CMC |
|---|---|---|---|---|
| | | % | % | % |
| A-Solution Mixture | | 50 | 3.325 | 0 |
| B-Solution Mixture | | 50 | 5.875 | 0.08 |
| Total | | 100 | 9.20 | 0.08 |

The cream cheese curd-like complex was then used to prepare a cream cheese product according to table 4 below. UF curd was prepared by culturing and fermenting a mixture of milk and and then using ultrafiltration to separate the curd from whey. UF curd, curd-like complex, cream, and buttermilk according to the recipe in Table 4 were mixed using a Lightning mixer (Grafton, Wisconsin). The pH was adjusted to pH 4.9 with a small amount of 44 percent lactic acid. The mixture was then heated to 60°C (140°F) in a Thermomix mixer. The mixture was homogenized at 5000/500 psi using a two-stage homogenizer. The remaining ingredients were added and the mixture was heated to 85°C (185°F) in the Thermomix. The temperature was held at 85°C (185°F) for at least 30 minutes. The mixture was then homogenized at 5000/500 psi and the samples were collected in 8 ounce plastic tubs. The tubs were then cooled and stored under refrigeration.

A control cream cheese product (without the curd-like complex) was prepared as described above according to the recipe in Table 4.

**Table 4.**

| **Cream Cheese Formula** | **Inventive** w/ curd-like complex | **Control** w/o curd-like complex |
|---|---|---|
| | % | % |
| Curd-like complex | 50.44 | 0 |
| UF curd | 30 | 30 |
| Cream | 14.55 | 13.58 |
| Water | 0 | 25.32 |
| Whole milk | 0 | 19.27 |
| Buttermilk | 3.18 | 8.11 |
| BiPro | 0 | 1.79 |
| 44% Lactic acid (gm) | 0.4 | 0.4 |
| Carrageenan | 0.4 | 0.43 |
| Xanthan | 0.25 | 0.25 |
| CMC | 0 | 0.03 |
| Salt | 0.73 | 0.77 |
| Sorbic acid | 0.05 | 0.05 |
| Total | 100 | 100 |

The resulting inventive and control cream cheese products had similar moisture, fat, protein, lactose, salt, and casein/whey content, as indicated in Table 5 below.

**Table 5.**

| **Cream Cheese Composition** | **Inventive** with curd-like complex | **Control** without curd-like complex |
|---|---|---|
| | % | % |
| Moisture | 72.67 | 72.76 |
| Fat | 10 | 10 |
| Protein | 8 | 8 |
| Lactose | 6.52 | 6.46 |
| Salt | 0.81 | 0.80 |
| Casein/Whey | 63/37 | 62/38 |
| | | |
| **Yield Stress (Pa)** | 3580 | 2928 |

The cream cheese sample made with the inventive curd-like complex was more than 20 percent firmer based on yield stress even though the control had very similar moisture, fat, protein, lactose, and salt content. An expert sensory panel verified the differences between the two cream cheese samples. In addition, most panelists indicated that the inventive sample was equal to or slightly creamier than the control in spite of its higher firmness which is known to detract from perceived creaminess.

Advantageously, the fat and protein content is lower than that typically found in "light" soft cream cheeses (i.e., under 3.4 g fat per 1 ounce serving). This example demonstrates that the inventive curd-like complex can be used to provide cream cheese flavor without requiring cultured flavors. This example further demonstrates that the inventive curd-like complex is an excellent texture builder in cream cheese systems.

### Example 8

Curd-like Complex as Texture Builder and Fat Mimetic in Low Fat Cream Cheese

Part I: A curd-like complex was prepared according to the following formula and procedure. Solution A was prepared by dissolving 7 percent whey protein isolate (Provon 90 from Glanbia Nutritionals) in DI water and acidifying to pH 3.5 using 88 percent lactic acid. Solution B was prepared by dissolving 0.16 percent carrageenan and 25 percent buttermilk solids (3.62% moisture, 96.38% solids, 7.03% fat, 33% protein, 50% lactose, 8% ash) in whole milk (87.4% moisture, 3.7% fat, 3.5% protein, 4.9% lactose, 0.7% ash) and adjusting the pH to pH 8.5 with 6N NaOH. Additional 88% lactic acid was added to Solution A as needed to target a final complex with a pH of 5.1.

Solution A was heated in a Thermomix (Vorwerk USA Co., Longwood, FL) and maintained at 82.2°C (180°F). Separately, solution B was heated to 76.7-82.2°C(170-180°F). Hot solution B was added to the Thermomix and maintained at 82.2°C (180°F) for a few minutes with gentle mixing to form a complex with a composition according to Table 6. The complex thus formed was a soft cream cheese curd-like mass. The complex was allowed to cool to room temperature and stored refrigerated prior to use for cream cheese preparation.

**Table 6.**

| **Complex Composition** | Amount | Protein | Carrageenan |
|---|---|---|---|
| | % | % | % |
| Solution A | 50 | 3.15 | 0 |
| Solution B | 50 | 5.88 | 0.16 |
| Total | 100 | 9.03 | 0.08 |

The curd-like mass comprised 78.3 percent moisture, 21.7 percent solids, 2.75 percent fat, 9.03 percent protein (with a casein/whey ratio of 52/48), 8.73 percent lactose, and 1.35 percent ash.

Part II: Three batches of light soft cream cheese were prepared according to the recipes provided in Table 7 below. UF curd was prepared by culturing and fermenting a mixture of milk and cream and then concentrating or removing excess water using ultrafiltration. UF curd, SMAR complex prepared as above, buttermilk, and whey protein isolate (Bipro 95 from Danisco Food International) were combined in a beaker and mixed using a Lightning mixer (Grafton, Wisconsin). The amount of cream used in each sample was varied in order to adjust the overall fat content in the finished cream cheese because butterfat (mainly from cream) is a very functional component of cream cheese. The pH of each mixture was adjusted to pH 4.9 with 44 percent lactic acid and heated in a Thermomix mixer to 60°C (140°F). The moisture content was checked and then adjusted as needed to the target value of about 71 percent. Each mixture was then homogenized at 5000/500 psi and the remaining ingredients were then added. Each mixture was then heated in a Thermomix mixer to 82.2°C (180° F) and held for at lease 15 minutes while covered to minimize water loss. The mixtures were then homogenized again at 5000/500 psi and collected into 8 ounce plastic tubs. The tubs were cooled and stored under refrigeration.

**Table 7.**

| **Cream Cheese Formula** | **Sample 1** | **Sample 2** | **Sample 3** |
|---|---|---|---|
| | % | % | % |
| UF Curd | 75.585 | 52.01 | 40.22 |
| SMAR Complex | 10 | 30 | 40 |
| Cream | 7.92 | 13.13 | 15.75 |
| Bipro 95 | 1.03 | 1.21 | 1.3 |
| Buttermilk | 3.89 | 2.05 | 1.12 |
| 44% lactic acid | 0.4 | 0.4 | 0.4 |
| Carrageenan GP911 | 0.3 | 0.3 | 0.3 |
| Xanthan Gum | 0.15 | 0.15 | 0.15 |
| Salt | 0.675 | 0.7 | 0.71 |
| Sorbic acid | 0.05 | 0.05 | 0.05 |
| Total | 100 | 100 | 100 |
| Yield Stress (Pa) | 1733 | 1773 | *2427* |

| **Cream Cheese Composition** | % | % | *%* |
|---|---|---|---|
| Moisture | 71.50 | 71.50 | *71.50* |
| Fat | 12.00 | 12.00 | *12.00* |
| Protein | 8 | 8 | *8* |
| Lactose | 5.87 | 6.03 | *6.12* |
| Salt | 0.80 | 0.80% | *0.80* |
| Casein/whey ratio | 66/34 | 58/42 | 54/46 |

Light soft cream cheese samples with identical gross composition (e.g., fat content) and with increasing level of cheese curd substitution with SMAR complex were found firmer and generally creamier. Sample 2 was identified by an expert sensory panel as the creamiest of the three samples. Although still considered very creamy, the relatively lower creaminess of Sample 3 (having the highest level of curd substitution) may be partially explained by the significantly (up to 37 percent) higher firmness of Sample 3. It was concluded that the SMAR complex is an exceptional texture/firmness builder while still a high degree of creaminess in high moisture, low fat, low protein cream cheese products.

### Example 9

Meat Protein Fiber. Mechanically separated turkey ("MST") was mixed with acidic water (pH 3.0) or basic water (pH 11) at a ratio of 1 part of MST and 4 parts of pH-adjusted water to extract the meat protein with maximum protein solubilization and minimum denaturation. Additional HCl or NaOH were added periodically as needed to maintain the initial pH for maximum extraction efficiency over a period of about 20 minutes. Each pH-adjusted mixture was then centrifuged at 3,000 rpm for 30 minutes and both fat and precipitate were removed to obtain acidic and basic protein-rich solutions. The acidic and basic protein-rich solutions were then mixed together in a 1:1 ratio and pH adjusted to form a mixture with a final equilibrated pH of about 5.5 in which the protein from both solutions was isoelectrically precipitated into a structureless, fine powder caught on a sieve.

The same experiment was repeated except that both acidic and basic protein solutions were first heated to 87.8°C (190°F) for 30 minutes before mixing together at 87.8°C (190°F). Upon mixing, both isoelectric precipitation and thermal crosslinking took place simultaneously to produce an insoluble protein mass having an unexpectedly large, coarse, fibrous structure.

### Example 10

Ground Turkey with Improved Water Holding Capacity. This example demonstrates the use of simultaneous acervation reactions to generate high volume, high moisture restructured turkey meat. A ground turkey emulsion ("emulsion A") was prepared by blending one portion of ground lean turkey meat with two portions of DI water. A portion of the emulsion was divided into emulsions A1 and A2. A portion of turkey emulsion A1 was adjusted to pH 3.0 with 5N HCl. A portion of emulsion A2 was adjusted to pH 11.0 with 5N NaOH. Three sets of experiments were conducted to demonstrate the importance of simultaneous reactions.

In the first experiment (hereinafter "control 1"), emulsion A was heated to 82.2°C (180°F) for 2 minutes. Control 1 demonstrated conventional thermal denaturation of a turkey emulsion.

In the second experiment (hereinafter "control 2"), equal amounts of emulsion A1 and emulsion A2 were mixed at room temperature and then heated to 180°F for 2 minutes. Control 2 demonstrated coacervation and thermal denaturation of turkey protein performed in sequence.

In the third experiment (hereinafter "inventive"), equal amounts of emulsion A1 and emulsion A2 were heated separately to 180°F and then mixed together and held for 2 minutes. This experiment demonstrated two reactions-coacervation and thermal denaturation-carried out simultaneously.

All experiments resulted in a final pH of 6.37 ± 0.07. The weight percent of meat retained on top of a US18 sieve was then measured. The results are summarized below in Table 8. The sample made from the inventive simultaneous reactions had a much higher drained weight compared to those made from thermal denaturation (control 1) and coacervation and thermal denaturation performed in sequence (control 2). As provided in Table 8 below, the percentages of drained weight for control 1, control 2, and inventive sample were 41.1 percent, 36.4 percent, and 50.1 percent, respectively. The inventive sample showed 18 percent improvement over control 1 and about 27 percent improvement over control 2.

**Table 8.**

| | **Control 1** | **Control 2** | **Inventive** |
|---|---|---|---|
| | Thermal Denaturation Only | Coacervation and Thermal Denaturation in Sequence | Simultaneous Coacervation and Thermal Denaturation |
| % of drained weight* | 41.1 | 36.4 | 50.1 |

| | | | |
|---|---|---|---|
| * weight percent of meat retained on top of sieve. | | | |

Numerous modifications and variations in practice of the processes described herein are expected to occur to those skilled in the art upon consideration of the foregoing detailed description. Consequently, such modifications and variations are intended to be included within the scope of the following claims.

## Claims

1. A method for producing a structured polymer matrix, the method comprising:
a-1) preparing at least one aqueous solution containing one or more food polymers, wherein the one or more food polymers are capable of undergoing at least two acervation mechanisms, and wherein conditions are such that the at least two acervation mechanisms are not activated prior to an activation step;
a-2) treating the at least one aqueous solution containing one or more food polymers to activate the at least two acervation mechanisms simultaneously; and
a-3) allowing the at least two activated acervation mechanisms to proceed until the structured polymer matrix is obtained,
or
b-1) preparing two or more aqueous polymer solutions, each solution comprising one or more edible polymers, wherein the one or more edible polymers in each solution are capable of undergoing at least one acervation mechanism, and wherein conditions in each solution are such that the at least one acervation mechanism is not activated in each solution; and
b-2) combining the two or more aqueous polymer solutions to provide conditions whereby two or more acervation mechanisms occur simultaneously,
or
c-1) preparing a positively charged aqueous solution having a pH of about 2 to about 5 and a temperature of at least about 71.1°C (160°F), wherein the solution comprises at least one food polymer capable of undergoing at least one acervation mechanism, wherein conditions in the solution are such that the at least one acervation is not activated;
c-2) preparing a negatively charged second aqueous polymer solution having a pH of about 8 to about 11 and a temperature of at least about 71.1°C (160°F), wherein the solution comprises at least one food polymer capable of undergoing at least one acervation mechanism, wherein conditions in the solution are such that the at least one acervation is not activated; and
c-3) combining the two heated polymer solutions to provide conditions whereby two or more acervation mechanisms occur simultaneously,
or
d-1) preparing a positively charged first aqueous polymer-containing solution having a pH of about 2 to about 5, wherein the solution comprises at least one food polymer capable of undergoing at least one acervation mechanism, wherein the pH is less than the isoelectric pH of the polymer and conditions in the solution are such that the at least one acervation mechanism is not activated;
d-2) preparing a negatively charged second aqueous polymer-containing solution having a pH of about 8 to about 11, wherein the solution comprises at least one food polymer capable of undergoing at least one acervation mechanism and conditions in the solution are such that the at least one acervation mechanism is not activated;
d-3) heating the first polymer solution to a temperature greater than or equal to the temperature at which the first polymer would form crosslinks at a pH less than about 1 pH unit lower than the isoelectric point of the first polymer;
d-4) heating the second polymer solution to a temperature greater than or equal to the temperature of the first polymer solution; and
d-5) combining the two heated polymer solutions to provide a final pH upon mixing such that coacervation and thermal crosslinking mechanisms occur simultaneously, or
e-1) preparing a positively charged first aqueous polymer solution having a pH of about to 2 to about 4, wherein the solution comprises at least one food polymer capable of undergoing at least one acervation mechanism and conditions in the solution are such that the at least one Acervation mechanism is not activated;
e-2) preparing a negatively charged second aqueous polymer solution having a pH of about 8 to about 10, wherein the solution comprises at least one food polymer capable of undergoing at least one acervation mechanism and conditions in the solution are such that the at least one acervation mechanism is not activated;
e-3) adding multivalent mineral cations to one of the first and second polymer solutions;
e-4) heating the first and second polymer solutions to a temperature of at least about 71.1°C (160°F); and
e-5) combining the two heated polymer solutions to provide a final pH such that simultaneous isoelectric precipitation occurs simultaneously with ionic precipitation.

2. The method of Claim 1, wherein the at least one aqueous solution prepared in step a-1) comprises at least one food polymer selected from the group consisting of proteins, polysaccharides, and mixtures thereof.

3. The method of Claim 1, wherein the at least one aqueous solution prepared in step a-1) comprises a food protein and an anionic polysaccharide.

4. The method of any one of Claims 1 to 3, wherein the at least two acervation mechanisms are selected from the group consisting of polymerization, thermal crosslinking, coacervation, chemical complexing, isoelectric precipitation, ionic precipitation, solvent precipitation, gelation, and denaturation.

5. The method of Claim 1, wherein each of the two or more polymer solutions prepared in step b-1) comprises at least one food polymer selected from the group consisting of proteins, polysaccharides, and mixtures thereof.

6. The method of Claims 1 or 5, wherein at least one of the polymer solutions prepared in step b-1) is prepared using a food ingredient from the group consisting of milk, cheese whey, egg, and meat slurry.

7. The method of any one of Claims 1, 5 or 6, wherein at least one of the polymer solutions prepared in step b-1) comprises a food protein and at least one of the polymer solutions comprises an anionic polysaccharide.

8. The method of Claim 1, wherein each of the first and second polymer solutions prepared in steps c-1) and c-2) comprises at least one food polymer selected from the group consisting of proteins and polysaccharides.

9. A structured polymer complex formed by a process comprising:
f-1) preparing at least one aqueous solution containing one or more food polymers, wherein the one or more food polymers are capable of undergoing at least two acervation mechanisms, and wherein conditions are such that the at least two acervation mechanisms are not activated;
f-2) treating the at least one aqueous solution to activate the at least two acervation mechanisms simultaneously; and
f-3) allowing the at least two activated acervation mechanisms to proceed until the structured polymer matrix is obtained, the structured polymer matrix having a structure different from that formed by the same acervation mechanisms performed individually or sequentially, or
g-1) preparing two or more aqueous polymer solutions, each solution comprising one or more edible polymers, wherein the one or more edible polymers in each solution are capable of undergoing at least one acervation mechanism, and wherein conditions in each solution are such that the at least one acervation mechanism is not activated in each solution; and
g-2) combining the two or more aqueous polymer solutions to provide conditions whereby two or more acervation mechanisms occur simultaneously, the structured polymer matrix having a structure different from that formed by the same acervation mechanisms performed individually or sequentially.

10. A food product comprising the structured polymer complex of claim 9.

## Patentansprüche

1. Verfahren zur Herstellung einer strukturierten Polymermatrix, wobei das Verfahren umfasst:
a-1) Zubereiten zumindest einer wässrigen Lösung enthaltend ein oder mehrere Lebensmittelpolymere, wobei das eine oder mehrere Lebensmittelpolymere in der Lage sind zumindest zwei Acervationsmechanismen zu durchlaufen und wobei die Bedingungen derart sind, dass die zumindest zwei Acervationsmechanismen nicht vor einem Aktivierungsschritt aktiviert werden;
a-2) Behandeln der zumindest einer wässrigen Lösung enthaltend ein oder mehrere Lebensmittelpolymere, um die zumindest zwei Acervationsmechanismen gleichzeitig zu aktivieren; und
a-3) Zulassen, dass die zumindest zwei aktivierten Acervationsmechanismen voranschreiten bis die strukturierte Polymermatrix erhalten ist,
oder
b-1) Zubereiten von zwei oder mehr wässrigen Polymerlösungen, jede Lösung dabei ein oder mehrere essbare Polymere enthaltend, wobei der eine oder mehrere essbare Polymere in jeder Lösung in der Lage sind zumindest einen Acervationsmechanismus zu durchlaufen und wobei die Bedingungen in jeder Lösung derart sind, dass der zumindest eine Acervationsmechanismus nicht in jeder Lösung aktiviert ist; und
b-2) Vereinigung der zwei oder mehrerer wässriger Polymerlösungen um Bedingungen zu erhalten bei denen zwei oder mehrere Acervationsmechanismen gleichzeitig stattfinden,
oder
c-1) Zubereiten einer positiv geladenen wässrigen Lösung mit einem pH von etwa 2 bis etwa 5 und einer Temperatur von zumindest etwa 71,1°C (160°F), wobei die Lösung zumindest ein Lebensmittelpolymer umfasst, welches in der Lage ist zumindest einen Acervationsmechanismus zu durchlaufen, wobei die Bedingungen der Lösung derart sind, dass die zumindest eine Acervation nicht aktiviert ist;
c-2) Zubereiten einer zweiten negativ geladenen wässrigen Polymerlösung mit einem pH von etwa 8 bis etwa 11 und einer Temperatur von zumindest etwa 71,1°C (160°F), wobei die Lösung zumindest ein Lebensmittelpolymer umfasst, welches in der Lage ist zumindest einen Acervationsmechanismus zu durchlaufen, wobei die Bedingungen in der Lösung derart sind, dass die zumindest eine Acervation nicht aktiviert ist; und
c-3) Vereinigung der zwei erhitzten Polymerlösungen, um Bedingungen zu erhalten bei denen zwei oder mehr Acervationsmechanismen gleichzeitig stattfinden,
oder
d-1) Zubereiten einer positiv geladenen ersten, wässrigen Polymerenthaltenden Lösung mit einem pH von etwa 2 bis etwa 5, wobei die Lösung zumindest ein Lebensmittelpolymer umfasst, welches in der Lage ist, zumindest einen Acervationsmechanismus zu durchlaufen, wobei der pH niedriger ist als der isoelektrische pH des Polymers und die Bedingungen der Lösung derart sind, dass der zumindest eine Acervationsmechanismus nicht aktiviert ist;
d-2) Zubereiten einer negativ geladenen zweiten, wässrigen Polymerenthaltenden Lösung mit einem pH von etwa 8 bis etwa 11, wobei die Lösung zumindest ein Lebensmittelpolymer umfasst, welches in der Lage ist, zumindest einen Acervationsmechanismus zu durchlaufen und die Bedingungen in der Lösung derart sind, dass der zumindest eine Acervationsmechanismus nicht aktiviert ist;
d-3) Erhitzen der ersten Polymerlösung auf eine Temperatur höher als oder gleich der Temperatur bei welcher das erste Polymer Querverbindungen ausbilden würde bei einem pH der weniger als etwa eine pH-Einheit niedriger liegt als der isoelektrische Punkt des ersten Polymers;
d-4) Erhitzen der zweiten Polymerlösung auf eine Temperatur höher als oder gleich der Temperatur der ersten Polymerlösung; und
d-5) Vereinigung der beiden erhitzten Polymerlösungen, um einen finalen pH durch die Vermischung zu erhalten derart, dass Koacervation und thermale Quervemetzungsmechanismen gleichzeitig stattfinden, oder
e-1) Zubereiten einer positiv geladenen ersten, wässrigen Polymerlösung mit einem pH von etwa 2 bis etwa 4, wobei die Lösung zumindest ein Lebensmittelpolymer umfasst, welches in der Lage ist, zumindest einen Acervationsmechanismus zu durchlaufen und Bedingungen in der Lösung derart sind, dass der zumindest eine Acervationsmechanismus nicht aktiviert ist;
e-2) Zubereiten einer negativ geladenen zweiten, wässrigen Polymerlösung mit einem pH von etwa 8 bis etwa 10, wobei die Lösung zumindest ein Lebensmittelpolymer umfasst, welches in der Lage ist, zumindest einen Acervationsmechanismus zu durchlaufen und Bedingungen in der Lösung derart sind, dass der zumindest eine Acervationsmechanismus nicht aktiviert ist;
e-3) Hinzufügen multivalenter Mineralkationen zu einer der ersten oder zweiten Polymerlösungen;
e-4) Erhitzen der ersten und zweiten Polymerlösungen auf eine Temperatur von mindestens etwa 71,1°C (160°F); und
e-5) Vereinigung der beiden erhitzten Polymerlösungen, um einen finalen pH zu erhalten derart, dass die simultane isoelektrische Fällung gleichzeitig mit der ionischen Fällung stattfindet.

2. Verfahren nach Anspruch 1, wobei die zumindest eine wässrige Lösung vorbereitet in Schritt a-1) zumindest ein Lebensmittelpolymer enthält ausgewählt aus der Gruppe bestehend aus Proteinen, Polysacchariden und Mischungen hiervon.

3. Verfahren nach Anspruch 1, wobei die zumindest eine wässrige Lösung vorbereitet in Schritt a-1) ein Lebensmittelprotein und ein anionisches Polysaccharid umfasst.

4. Verfahren nach einem der Ansprüche 1 bis 3, wobei die zumindest zwei Acervationsmechanismen ausgewählt sind aus der Gruppe bestehend aus Polymerisation, thermischer Vernetzung, Koacervation, chemischer Komplexbildung, isoelektrischer Fällung, ionischer Fällung, Lösemittelfällung, Gelierung und Denaturierung.

5. Verfahren nach Anspruch 1, wobei jedes der zwei oder mehr Polymerlösungen zubereitet in Schritt b-1) zumindest ein Lebensmittelpolymer umfasst ausgewählt aus der Gruppe bestehend aus Proteinen, Polysacchariden und Mischungen hiervon.

6. Verfahren nach Anspruch 1 oder 5, wobei zumindest eine der Polymerlösungen zubereitet im Schritt b-1) zubereitet wird unter Benutzung eines Nahrungsmittelbestandteils aus der Gruppe bestehend aus Milch, Käsemolke, Ei und Fleischbrei.

7. Verfahren nach einem der Ansprüche 1, 5 oder 6, wobei zumindest eine der Polymerlösungen zubereitet im Schritt b-1) ein Lebensmittelprotein umfasst und zumindest eine der Polymerlösungen ein anionisches Polysaccharid umfasst.

8. Verfahren nach Anspruch 1, wobei jeder der ersten und zweiten Polymerlösungen zubereitet in Schritten c-1) und c-2) zumindest ein Lebensmittelpolymer umfasst ausgewählt aus der Gruppe bestehend aus Proteinen und Polysacchariden.

9. Strukturierter Polymerkomplex der durch ein Verfahren gebildet wird umfassend:
f-1) Zubereiten zumindest einer wässrigen Lösung enthaltend ein oder mehrere Lebensmittelpolymere, wobei dass eine oder mehrere Lebensmittelpolymere in der Lage sind zumindest zwei Acervationsmechanismen zu durchlaufen und wobei die Bedingungen derart sind, dass die zumindest zwei Acervationsmechanismen nicht aktiviert sind;
f-2) Behandeln der zumindest einen wässrigen Lösung, um die zumindest zwei Acervationsmechanismen gleichzeitig zu aktivieren; und
f-3) Zulassen, dass die zumindest zwei aktivierten Acervationsmechanismen voranschreiten bis eine strukturierte Polymermatrix erhalten ist, die strukturierte Polymermatrix eine Struktur aufweist, anders als solche, die durch die gleichen Acervationsmechanismen gebildet wird wenn diese einzeln oder nacheinander ausführt werden, oder
g-1) Zubereiten von zwei oder mehreren wässrigen Polymerlösungen, jede der Lösungen ein oder mehrere essbare Polymere umfassend, wobei das eine oder mehrere essbare Polymere in jeder der Lösungen in der Lage sind, zumindest einen Acervationsmechanismus zu durchlaufen und wobei die Bedingungen in jeder Lösung derart sind, dass der zumindest eine Acverationsmechanismus nicht in jeder Lösung aktiviert ist; und
g-2) Vereinigen der zwei oder mehrerer wässriger Polymerlösungen um Bedingungen zu erhalten, bei denen zwei oder mehrere Acervationsmechanismen gleichzeitig stattfinden, die strukturierte Polymermatrix eine Struktur aufweist anders als solche, die durch die gleichen Acervationsmechanismen gebildet wird wenn diese einzeln oder nacheinander ausgeführt werden.

10. Lebensmittelprodukt umfassend den strukturierten Polymerkomplex gemäß Anspruch 9.

## Revendications

1. Procédé de fabrication d'une matrice polymère structurée, le procédé comprenant :
a-1) la préparation d'au moins une solution aqueuse contenant un ou plusieurs polymères alimentaires, le ou les polymères alimentaires étant capables de subir au moins deux mécanismes d'acervation, et les conditions étant telles que les au moins deux mécanismes d'acervation ne sont pas activés avant une étape d'activation ;
a-2) le traitement de l'au moins une solution aqueuse contenant un ou plusieurs polymères alimentaires pour activer les au moins deux mécanismes d'acervation simultanément ; et
a-3) la poursuite des au moins deux mécanismes d'acervation activés jusqu'à ce que la matrice polymère structurée soit obtenue, ou
b-1) la préparation de deux solutions polymères aqueuses ou plus, chaque solution comprenant un ou plusieurs polymères comestibles, le ou les polymères comestibles dans chaque solution étant capables de subir au moins un mécanisme d'acervation, et les conditions dans chaque solution étant telles que l'au moins un mécanisme d'acervation n'est pas activé dans chaque solution ; et
b-2) la combinaison des deux solutions polymères aqueuses ou plus pour obtenir des conditions dans lesquelles deux ou plusieurs mécanismes d'acervation se produisent simultanément, ou
c-1) la préparation d'une solution aqueuse à charge positive ayant un pH d'environ 2 à environ 5 et une température d'au moins environ 71,1 °C (160 °F), la solution comprenant au moins un polymère alimentaire capable de subir au moins un mécanisme d'acervation, les conditions dans la solution étant telles que l'au moins une acervation n'est pas activée ;
c-2) la préparation d'une seconde solution polymère aqueuse à charge négative ayant un pH d'environ 8 à environ 11 et une température d'au moins environ 71,1 °C (160 °F), la solution comprenant au moins un polymère alimentaire capable de subir au moins un mécanisme d'acervation, les conditions dans la solution étant telles que l'au moins une acervation n'est pas activée ; et
c-3) la combinaison des deux solutions polymères chauffées pour obtenir des conditions dans lesquelles deux ou plusieurs mécanismes d'acervation se produisent simultanément, ou
d-1) la préparation d'une première solution contenant du polymère aqueuse à charge positive ayant un pH d'environ 2 à environ 5, la solution comprenant au moins un polymère alimentaire capable de subir au moins un mécanisme d'acervation, le pH étant inférieur au pH isoélectrique du polymère et les conditions dans la solution étant telles que l'au moins un mécanisme d'acervation n'est pas activé ;
d-2) la préparation d'une seconde solution contenant du polymère aqueuse à charge négative ayant un pH d'environ 8 à environ 11, la solution comprenant au moins un polymère alimentaire capable de subir au moins un mécanisme d'acervation et les conditions dans la solution étant telles que l'au moins un mécanisme d'acervation n'est pas activé ;
d-3) le chauffage de la première solution polymère à une température supérieure ou égale à la température à laquelle le premier polymère formerait des réticulations à un pH inférieur à environ 1 unité de pH inférieure au point isoélectrique du premier polymère ;
d-4) le chauffage de la seconde solution polymère à une température supérieure ou égale à la température de la première solution polymère ; et
d-5) la combinaison des deux solutions polymères chauffées pour obtenir un pH final lors du mélange tel que les mécanismes de coacervation et de réticulation thermique se produisent simultanément, ou
e-1) la préparation d'une première solution polymère aqueuse à charge positive ayant un pH d'environ 2 à environ 4, la solution comprenant au moins un polymère alimentaire capable de subir au moins un mécanisme d'acervation et les conditions dans la solution étant telles que l'au moins un mécanisme d'acervation n'est pas activé ;
e-2) la préparation d'une seconde solution polymère aqueuse à charge négative ayant un pH d'environ 8 à environ 10, la solution comprenant au moins un polymère alimentaire capable de subir au moins un mécanisme d'acervation et les conditions dans la solution étant telles que l'au moins un mécanisme d'acervation n'est pas activé ;
e-3) l'ajout de cations minéraux multivalents à une des première et seconde solutions polymère ;
e-4) le chauffage des première et seconde solutions polymères à une température d'au moins environ 71,1 °C (160 °F), et
e-5) la combinaison des deux solutions polymères chauffées pour obtenir un pH final tel qu'une précipitation isoélectrique se produit simultanément avec une précipitation ionique.

2. Procédé selon la revendication 1, l'au moins une solution aqueuse préparée à l'étape a-1) comprenant au moins un polymère alimentaire choisi dans le groupe constitué des protéines, des polysaccharides et de mélanges de celles-ci.

3. Procédé selon la revendication 1, l'au moins une solution aqueuse préparée à l'étape a-1) comprenant une protéine alimentaire et un polysaccharide anionique.

4. Procédé selon l'une quelconque des revendications 1 à 3, les au moins deux mécanismes d'acervation étant choisis dans le groupe constitué de la polymérisation, de la réticulation thermique, de la coacervation, de la complexion chimique, de la précipitation isoélectrique, de la précipitation ionique, de la précipitation dans un solvant, de la gélification et de la dénaturation.

5. Procédé selon la revendication 1, chacune des deux solutions polymères ou plus préparées à l'étape b-1) comprenant au moins un polymère alimentaire choisi dans le groupe constitué des protéines, des polysaccharides et de mélanges de celles-ci.

6. Procédé selon les revendications 1 ou 5, au moins une des solutions polymères préparées à l'étape b-1) étant préparée en utilisant un ingrédient alimentaire choisi dans le groupe constitué du lait, du lactosérum de fromage, de l'oeuf et d'une bouillie de viande.

7. Procédé selon l'une quelconque des revendications 1, 5 ou 6, au moins une des solutions polymères préparées à l'étape b-1) comprenant une protéine alimentaire et au moins une des solutions polymères comprenant un polysaccharide anionique.

8. Procédé selon la revendication 1, chacune des première et seconde solutions polymères préparées aux étapes c-1) et c-2) comprenant au moins un polymère alimentaire choisi dans le groupe constitué des protéines et des polysaccharides.

9. Complexe polymère structuré formé par un procédé comprenant :
f-1) la préparation d'au moins une solution aqueuse contenant un ou plusieurs polymères alimentaires, le ou les polymères alimentaires étant capables de subir au moins deux mécanismes d'acervation, et les conditions étant telles que les au moins deux mécanismes d'acervation ne sont pas activés ;
f-2) le traitement de l'au moins une solution aqueuse pour activer les au moins deux mécanismes d'acervation simultanément ; et
f-3) la poursuite des au moins deux mécanismes d'acervation activés jusqu'à ce que la matrice polymère structurée soit obtenue, la matrice de polymère structurée ayant une structure différente de celle formée par les mêmes mécanismes d'acervation effectuée de manière individuelle ou en séquence, ou
g-1) la préparation de deux ou plusieurs solutions polymères aqueuses, chaque solution comprenant un ou plusieurs polymères comestibles, le ou les polymères comestibles dans chaque solution étant capables de subir au moins un mécanisme d'acervation, et les conditions dans chaque solution étant telles que l'au moins un mécanisme d'acervation n'est pas activé dans chaque solution ; et
g-2) la combinaison des deux solutions polymères aqueuses ou plus pour obtenir des conditions dans lesquelles deux mécanismes d'acervation ou plus se produisent simultanément, la matrice polymère structurée ayant une structure différente de celle formée par les mêmes mécanismes d'acervation effectués de manière individuelle ou en séquence.

10. Produit alimentaire comprenant le complexe polymère structuré de la revendication 9.
